(19) Europäisches Patentamt
European Patent Office
Office européen des brevets

(11) EP 0 796 861 B1

(12) FASCICULE DE BREVET EUROPEEN

(45) Date de publication et mention de la délivrance du brevet:
**08.04.1998 Bulletin 1998/15**

(51) Int Cl.[6]: **C07F 7/21**, C07F 7/08, C08G 77/38, A61K 31/695

(21) Numéro de dépôt: **97400432.7**

(22) Date de dépôt: **26.02.1997**

# (54) Nouveaux dérivés siliciés de l'acide salicilique à propriétés desquamantes

Neue Siliciumhaltige Salicylsäurederivate mit abschuppenden Eigenschaften

Silated derivatives of salicilic acid with desquamative properties

(84) Etats contractants désignés:
**AT BE CH DE ES FR GB IT LI NL SE**

(30) Priorité: **22.03.1996 FR 9603622**

(43) Date de publication de la demande:
**24.09.1997 Bulletin 1997/39**

(73) Titulaire: **L'OREAL**
**75008 Paris (FR)**

(72) Inventeurs:
- **Richard, Hervé**
  **93420 Villepinte (FR)**
- **Leduc, Madeleine**
  **Rue des Boulets, 75011 Paris (FR)**
- **Lagrange, Alain**
  **77450 Coupvray (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
**FR-A- 2 683 455**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 796 861 B1

## Description

La présente invention a pour objet de nouveaux composés du type organosiloxanes et organosilanes, présentant pour caractéristique commune d'avoir tous au moins une fonction dérivée de l'acide salicylique. La présente invention a également pour objet l'utilisation de ces nouveaux dérivés dans des compositions topiques comme produits desquamantes et/ou pour lutter contre le vieillissement, ainsi que les compositions topiques contenant ces produits, et un procédé de traitement non thérapeutique de la peau.

On recherche en cosmétique des produits ayant une activité desquamante, notamment dans les produits anti-pelliculaires, les compositions de traitement des peaux sèches, les masques de beauté et pour les procédés dits de «peeling». Ces produits, tout en permettant l'élimination des peaux mortes ou hyperkératinisées, ne doivent pas entraîner d'inflammation.

En dermopharmacie, l'utilisation des produits desquamantes et comédolytiques présente également un grand intérêt, notamment dans le traitement des maladies affectant la couche cornée de l'homme ou de l'animal, telles que les verrues, l'acné, l'eczema, le psoriasis, les ulcères, etc...

L'acide salicylique est connu pour ses propriétés desquamantes et il est généralement utilisé comme agent desquamant contre l'acné. Certains de ses dérivés sont également connus pour cette activité, on pourra se reporter en particulier au document FR-A-2581 542.

D'autre part, on constate, au cours du processus de vieillissement, l'apparition de différents signes au niveau de la peau, très caractéristiques de ce vieillissement, se traduisant notamment par une modification de la structure et de la fonction cutanée. Ces signes sont particulièrement marqués sur les zones découvertes, telles que le visage, les mains, auxquelles se rajoutent généralement des caractéristiques particulières dues à l'exposition de la peau à la lumière solaire (vieillissement actinique).

Le vieillissement cutané résultant d'effets sur la peau de facteurs intrinsèques ou extrinsèques, se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement «normal» lié à l'âge, alors que d'autres sont plus spécifiques du vieillissement extrinsèque, c'est-à-dire du vieillissement provoqué d'une manière générale par l'environnement; il s'agit plus particulièrement du photo-vieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une accumulation excessive de matière élastique dans le derme supérieur et une dégénérescence des fibres de collagène.

D'après l'état de l'art, dont on peut citer les documents EP-A-378936 et WO-93/10756, l'acide salicylique et certains de ses dérivés peuvent être utilisés pour traiter le vieillissement de la peau.

Cependant, certaines de ces substances ne présentent pas toutes les propriétés requises pour une utilisation convenable dans les compositions cosmétiques et/ou dermatologiques. En particulier, leur solubilité dans les différents types de formulations utilisés n'est pas toujours suffisamment bonne (liposolubilité notamment) et elles peuvent également présenter une mauvaise résistance à l'eau et à la sueur. Il est également souhaitable que ces substances ne pénètrent pas dans la peau.

Ainsi, on a cherché à obtenir des produits à propriétés améliorées par rapport aux produits de l'état de la technique, en particulier au niveau de leur liposolubilité et de leur caractère cosmétique, en venant fixer par greffage (hydrosilylation) un groupement acide salicylique sur une chaîne macromoléculaire de type silicone (organopolysiloxane).

On connait par de nombreux documents (FR-A-1,124,824, GB-A-1,164,522, JP-A-71,002,575, FR-A-2,200,275, FR-A-2,550,787, EP-A-138,321, EP-A-138,590), des organopolysiloxanes greffés par des dérivés de l'acide salicylique. Toutefois, dans tous ces documents, le dérivé de l'acide salicylique est relié au polymère siloxane sous la forme d'un radical salicylate ou salicylate d'alcoyle et aucun de ces documents ne suggère d'utiliser de tels polymères pour la desquamation de la peau.

On connait, par le document FR-A-2,683,455, l'utilisation de monométhyltrisilanol d'acide salicylique pour la stimulation de la synthèse localisée des tissus conjonctifs.

Aussi, c'est avec étonnement que la demanderesse a découvert de nouveaux dérivés siliciés de l'acide salicylique, ayant des propriétés desquamantes et permettant de traiter le vieillissement cutané, tout en étant doués d'une lipophilie qui facilite leur mise en oeuvre dans des compositions cosmétiques de forme galénique quelconque.

La présente invention a ainsi pour premier objet de nouveaux dérivés siliciés de l'acide salicylique répondant à l'une des formules (1) à (3) suivantes :

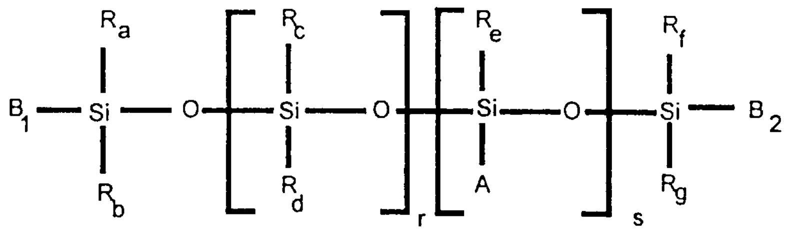

(1)

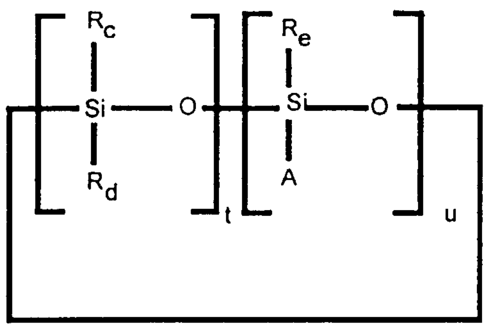

(2)

$$A\text{-}SiR'_1R'_2R'_3 \quad (3)$$

dans lesquelles :

- Ra à Rg, identiques ou différents, sont choisis parmi le groupe constitué des radicaux alcoyles en $C_1$-$C_{10}$ et alcényles en $C_2$-$C_{10}$, linéaires ou ramifiés, saturés ou insaturés, le radical phényle et le radical trifluoro-3,3,3 propyle, au moins 80% en nombre des radicaux Ra à Rg étant le radical méthyle.

- A désigne un radical monovalent lié directement à un atome de silicium et répondant à la formule (4) :

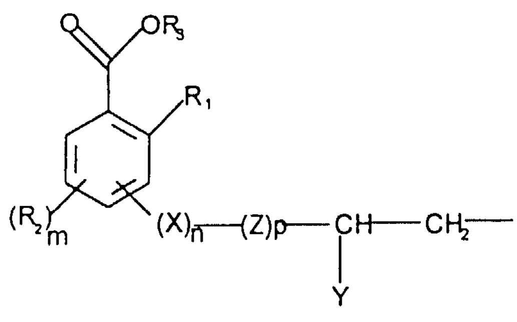

(4)

dans laquelle :

$R_1$ représente OH, un radical alcoxyle en $C_1$-$C_4$, linéaire ou ramifié, saturé ou insaturé, ou une fonction acyloxy de formule $O(C{=}O)R_4$, dans laquelle $R_4$ représente un radical alcoyle en $C_1$-$C_8$ ou alcényle en $C_2$-$C_8$, linéaire ou ramifié,

$R_2$, identiques ou différents, représentent un radical OH, alcoyle en $C_1$-$C_8$ ou alcényle en $C_2$-$C_8$, linéaire ou ramifié, alcoxyle en $C_1$-$C_8$, linéaire ou ramifié, deux $R_2$ adjacents pouvant former ensemble un groupement alcanedioxy- dans lequel la chaîne alcane est constituée de 1 ou 2 atomes de carbone,
$R_3$ représente un radical choisi parmi : un atome d'hydrogène, un cation pharmaceutiquement acceptable, un radical alcoyle en $C_1$-$C_6$ ou alcényle en $C_2$-$C_6$, linéaire ou ramifié et un radical benzyle, éventuellement substitué par un groupement choisi parmi les radicaux : alcoyle en $C_1$-$C_6$ ou alcényle en $C_2$-$C_6$, linéaire ou ramifié, hydroxy, amino, alcoxy en $C_1$-$C_6$ ou alcényloxy en $C_2$-$C_6$, linéaire ou ramifié, halogéno, acide carboxylique, carboxylate d'alcoyle en $C_1$-$C_6$ ou carboxylate d'alcényle en $C_2$-$C_6$, linéaire ou ramifié,
lorsque $R_1$ est différent de OH, $R_3$ est H ou un cation pharmaceutiquement acceptable,
m est choisi parmi les entiers 0, 1 ou 2,
p est choisi parmi les entiers 0 et 1,
X représente O, NH , C=O , NH(C=O)NH, NH(C=O) ou (C=O)NH,
n est choisi parmi les entiers 0 ou 1,
Z est un radical alcane di-yle en $C_1$-$C_6$ linéaire ou ramifié, saturé ou insaturé, éventuellement subtitué par un radical hydroxyle ou alcoxyle en $C_2$-$C_8$, linéaire ou ramifié, saturé ou insaturé,
Y représente un atome d'hydrogène, un radical hydroxyle ou un radical alcoxyle en $C_2$-$C_8$, linéaire ou ramifié, saturé ou insaturé,

- $B_1$ et $B_2$, identiques ou différents, sont choisis parmi les radicaux Ra à Rg et A définis ci-dessus,
- $R'_1$, $R'_2$, $R'_3$, identiques ou différents, sont choisis parmi les radicaux alcoyles en $C_1$-$C_8$ et alcényles en $C_2$-$C_8$, linéaires ou ramifiés, saturés ou insaturés, le radical phényle, et les radicaux alcoxyles en $C_1$-$C_4$, linéaires ou ramifiés, saturés ou insaturés,
- r est un nombre entier allant de 0 à 50,
- s est un nombre entier allant de 0 à 20, étant entendu que si s est nul, alors au moins l'un des radicaux $B_1$ et $B_2$ désigne le radical A,
- u est un nombre entier allant de 1 à 6, t est un nombre entier allant de 0 à 10, étant entendu que t+u est supérieur ou égal à 3,

Ces produits présentent par rapport à l'art antérieur des liposolubilités supérieures permettant de les introduire dans des compositions grasses, évitant un desèchement trop important de la peau du fait de l'activité desquamante.

Dans les formules (1) à (3) ci-dessus, A représente donc le groupement dérivé de l'acide salicylique qui, après fixation sur la chaîne siliconée de départ ou sur le silane de départ, confère aux composés de type organosiloxanes linéaires (formule (1)) ou cycliques (formule (2)) ou de type triorganosilanes (formule (3)) des propriétés desquamantes et de rajeunissement de la peau.

Comme cela ressort de la formule (4) donnée ci-dessus, l'accrochage du chaînon $-(X)_n$-Z-sur le noyau aromatique du motif salicylique, qui assure donc le raccordement dudit motif salicylique à l'atome de silicium de la chaîne siliconée ou du silane, peut, selon la présente invention, se faire dans toutes les positions disponibles offertes par le noyaux aromatique.

De préférence, cet accrochage se fait en position 3, 4 ou 5.

De même, l'accrochage du motif subsituant $R_2$ peut se faire dans toutes les autres positions disponibles au sein du noyau salicylique.

Dans les formules (1) à (3) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, iso-amyle, néo-pentyle, n-hexyle, n-heptyle, n-octyle, éthyl-2 hexyle et ter-octyle. De préférence, les radicaux Ra à Rg sont choisis parmi les radicaux méthyle, éthyle, propyle, n-butyle, n-octyle et éthyl-2 hexyle, et encore plus préférentiellement, les radicaux Ra à Rg représentent le méthyle.

Avantageusement, les radicaux $R'_1$, $R'_2$, $R'_3$ sont choisis parmi les radicaux méthyle, éthyle, propyle, n-butyle, n-octyle et éthyl-2 hexyle, et de preférence, $R'_1$, $R'_2$, $R'_3$ représentent le méthyle.

Selon un mode préféré de réalisation de l'invention, les radicaux alkyles $B_1$ et $B_2$ sont choisis parmi les radicaux méthyle, éthyle, propyle, n-butyle, n-octyle et éthyl-2 hexyle, et de préférence, les radicaux $B_1$ et $B_2$ représentent tous deux le méthyle.

Parmi les composés de formules (1) à (3) ci-dessus, on préfère mettre en oeuvre ceux répondant à la formule (1) ou à la formule (3), c'est-à-dire des organosiloxanes à chaine courte, linéaire ou les triorganosilanes.

Parmi les organosiloxanes linéaires faisant partie de la présente invention, on préfère plus particulièrement les

dérivés statistiques ou bien définis à blocs et présentant en plus notamment, au moins l'une, et encore plus préférentiellement l'ensemble, des caractéristiques suivantes :

- Ra à Rg sont des radicaux alcoyle et encore plus préférentiellement représentent le méthyle,
- $B_1$ et $B_2$ sont des radicaux alcoyle et encore plus préférentiellement méthyle (cas des composés linéaires de formule (1)),
- r est compris entre 0 et 3 inclusivement ; s est compris entre 0 et 3 inclusivement (cas des composés linéaires de formule (1)),
- $R_1$ est OH,
- $R_3$ est l'atome d'hydrogène,
- m = 0,
- n=0 ou X représente O (n=1).

Plus particulièrement, l'invention a pour objet les produits suivants :

- le 2-hydroxy-4-(3-triméthylsilanyl-propyloxy)-benzoate de méthyle,
- l'acide 2-hydroxy-4-(3-triméthylsilanyl-propyloxy)-benzoique,
- le 2-hydroxy-5-(3-triméthylsilanyl-propyloxy)-benzoate de méthyle,
- l'acide 2-hydroxy-5-(3-triméthylsilanyl-propyloxy)-benzoique,
- le 2-hydroxy-4-[2-méthyl-3-[1,3,3,3-tétraméthyl-1-[(triméthylsilyl)oxy]-disiloxanyl]-propyloxy]benzoate de méthyle,
- le 2-hydroxy-3-[2-méthyl-3-[1,3,3,3-tétramélhyl-1-[(triméthylsilyl)oxy]-disiloxanyl)-propyl]benzoate de méthyle,
- l'acide 2-hydroxy-4-[2-méthyl-3-[1,3,3,3-tétraméthyl-1-[(triméthylsilyl)oxy]-disiloxanyl]propyl]-benzoique,
- l'acide 2-hydroxy-3-[2-méthyl-3-[1,3,3,3-tétraméthyl-1-[(triméthylsilyl)oxy]-disiloxanyl]propyl]-benzoique,
- l'acide 2-hydroxy-5-[3-[1'3,3,3-tétraméthyl-1-[(triméthylsilyl)oxyldisiloxanyl]-propylamido]-benzoique.

Pour préparer les salicylates siliconés de formules (1), (2) et (3), on peut procéder classiquement en mettant en oeuvre une réaction d'hydrosylilation, à savoir:

$$\equiv Si\text{-}H + CH_2{=}CY\text{-} \rightarrow \equiv Si\text{-}CH_2\text{-}CHY\text{-}$$

à partir de la silicone ou du silane correspondant, dans lequel tous les radicaux A sont des atomes d'hydrogène. Cette silicone de départ est dénommée par la suite dérivé à SiH ; les groupes SiH peuvent être présents dans la chaîne et/ou aux extrémités de la chaîne silicone. Ces dérivés à SiH sont des produits bien connus dans l'industrie des silicones et sont généralement disponibles dans le commerce. Ils sont par exemple décrits dans les brevets américains US-A-3 220 972, US-A-3 697 473 et US-A-4 340 709.

Ce dérivé à SiH peut être donc représenté soit par la formule (1bis) suivante :

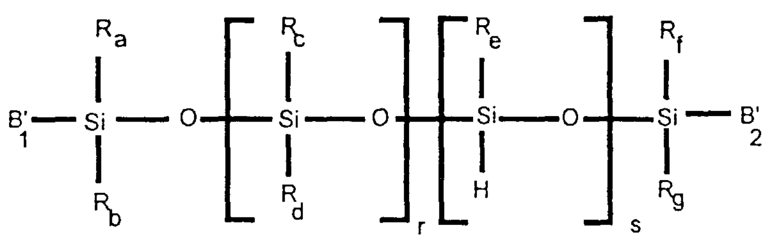

**(1 bis)**

dans laquelle Ra à Rg, r et s ont la signification donnée ci-dessus pour la formule (1) et les radicaux $B'_1$, $B'_2$, identiques ou différents, sont choisis parmi les radicaux Ra à Rg et un atome d'hydrogène,
soit par la formule (2bis) suivante :

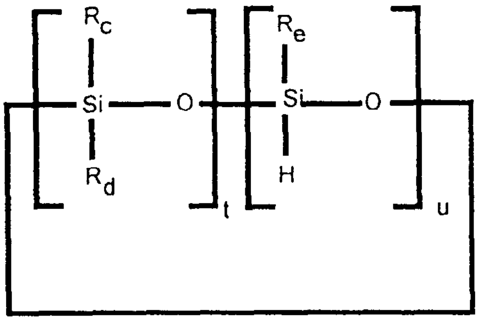

(2bis)

dans laquelle Rc à Re, t et u ont la signification donnée ci-dessus pour la formule (2). soit par la formule (3bis) suivante

$$HSiR'_1R'_2R'_3 \qquad (3bis)$$

dans laquelle $R'_1$, $R'_2$, $R'_3$ ont la même signification que dans la formule (3).

Sur ce dérivé à SiH de formule (1bis) (2bis) ou (3bis), on effectue donc une réaction d'hydrosilylation classique, opérée en présence d'une quantité catalytiquement efficace d'un catalyseur au platine, sur un dérivé organique d'acide salicylique de formule (4bis) suivante :

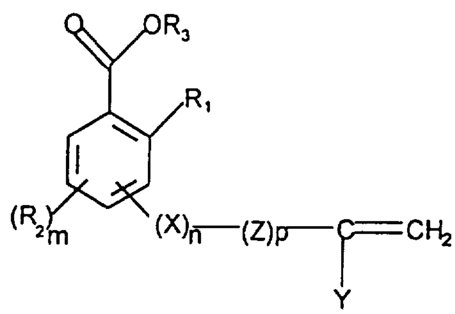

**(4bis)**

dans laquelle $R_1$, $R_2$, X, Z, Y, n, m et p ont la signification donnée ci-dessus pour la formule (4) et $R_3$ représente un radical choisi parmi : un radical alcoyle en $C_1$-$C_6$ ou alcényle en $C_2$-$C_6$, linéaire ou ramifié et un radical benzyle.

Les produits répondant aux formules (1), (2) et (3) dans lesquelles $R_3$ est l'atome d'hydrogène sont préparés à partir des dérivés de même formule dans lesquels :

$R_3$ est un radical alcoyle, par hydrolyse basique en milieu alcoolique,
ou
$R_3$ est un radical benzyle, par hydrogénation catalytique en présence d'un catalyseur du type palladium sur charbon, soit avec un agent de transfert d'hydrogène tel que le cyclohexène.

Des procédés convenant à la préparation des produits de formule (4bis) ci-dessus sont notamment décrits dans les brevets US- 4 316 033 et US- 4 328 346.

En outre, les détails des conditions opératoires à suivre pour conduire la réaction d'hydrosylilation entre les composés de formule (1bis) ou (2bis) ci-dessus avec le composé de formule (4bis) ci-dessus sont donnés dans la demande de brevet EP-A-0 392 883 précitée.

La présente invention a également pour objet l'utilisation des dérivés siliciés de l'acide salicylique répondant à l'une des formules (1) à (3), dans ou pour la fabrication d'une composition cosmétique ou dermatologique pour favoriser la desquamation de la peau humaine et/ou stimuler le renouvellement épidermique.

La présente invention a également pour objet l'utilisation des dérivés siliciés de l'acide salicylique répondant à l'une des formules (1) à (3), pour traiter le vieillissement intrinsèque et extrinsèque de la peau humaine.

La présente invention a également pour objet l'utilisation des dérivés siliciés de l'acide salicilyque répondant à l'une des formules (1) à (3), dans ou pour la fabrication d'une composition cosmétique ou dermatologique, notamment

pour lutter contre les rides et/ou les ridules et/ou les taches actiniques et/ou les dyschromies cutanées et/ou les dermites et/ou les cicatrices de la peau humaine.

En outre, l'invention a également pour objet l'utilisation des dérivés siliciés de l'acide salicilyque répondant à l'une des formules (1) à (3), dans ou pour la fabrication d'une composition cosmétique ou dermatologique anti-bactérienne, anti-solaire et/ou de protection de la peau humaine contre les radicaux libres.

Un test *in vitro* d'efficacité de la desquamation a été réalisé sur kératinocytes en utilisant de l'acide n-octanoyl-5-salicylique (composé $C_1$), l'acide 2-hydroxy-4-(3-triméthylsilanylpropyloxy)-benzoique (composé $C_2$), de l'acide 2-hydroxy-5-(3-triméthylsilanylpropyloxy)-benzoique (composé $C_3$).

Le principe du test repose sur le fait que la desquamation induit la libération de cornéocytes. Le pouvoir de desquamation du produit testé va être d'autant plus grand que le nombre de cornéocytes libérés sera important.

Le protocole du test a été le suivant : à partir de biopsies de peau, on a obtenu, par séparation de l'épiderme, des kératinocytes que l'on a dissociés par action enzymatique à la trypsine et mis en culture à la concentration de $2.10^5$ cellules/ml. La croissance et la différenciation des kératinocytes ont été obtenues par culture durant 10 à 20 jours en milieu spécifique.

Puis, après élimination du milieu de culture, on a ajouté le produit à tester et évalué l'activité du produit. Pour ce faire, on a réalisé deux prélèvements à $T_0$ et $T_{60}$, c'est-à-dire avant l'ajout du produit et 60 minutes après cet ajout, et on a analysé les prélèvements ainsi effectués au cytomètre de flux pour dénombrer la population de cornéocytes. Au cytomètre de flux, les populations de cornéocytes et de kératinocytes sont différenciées par traitement à l'acridine orange spécifique de l'ADN des cellules, qui se lie au noyau des cellules et révèle donc exclusivement la présence des kératinocytes.

L'indice de détachement cellulaire est déterminé par la différence entre $T_{60}$ et $T_0$.

La même mesure a été réalisée pour un témoin ne contenant pas de produit à tester car l'expérience produit inévitablement la libération de cornéocytes, même en l'absence d'actif. La variation du témoin a fixé arbitrairement la norme de 100 %.

Les résultats sont rassemblés dans le tableau ci-dessous :

| Témoin | Composé $C_1$ | Composé $C_2$ | Composé $C_3$ |
|---|---|---|---|
| 0 % | 103 % | 157 % | 135 % |

Ces résultats montrent clairement que l'acide 2-hydroxy-4-(3-triméthylsilanyl-propyloxy)-benzoique et l'acide 2-hydroxy-5-(3-triméthylsilanyl-propyloxy)-benzoique, à concentration égale à celle de l'acide n-octanoyl-5-salicylique connu comme étant un actif desquamant puissant, sont beaucoup plus actifs que celui-ci.

L'invention a encore pour objet un procédé de traitement non thérapeutique de la peau destiné à la desquamation de la peau humaine consistant à appliquer sur cette peau une composition contenant au moins un dérivé silicié de l'acide salicylique répondant à l'une des formules (1), (2) ou (3) dans un milieu cosmétiquement et/ou dermatologiquement acceptable.

L'invention a aussi pour objet un procédé de traitement non thérapeutique du vieillissement de la peau, consistant à appliquer sur la peau humaine une composition contenant au moins un dérivé silicié de l'acide salicylique tel que défini ci-dessus, dans un milieu cosmétiquement et/ou dermatologiquement acceptable.

L'invention a également pour objet des compositions cosmétiques et/ou dermatologiques caractérisées en ce qu'elles comprennent au moins un dérivé silicié de l'acide salicylique répondant à l'une des formules (1) à (3).

La composition de l'invention contient un milieu cosmétiquement ou dermatologiquement acceptable, c'est-à-dire un milieu compatible avec la peau, les ongles, les muqueuses, les tissus et les cheveux. La composition comprenant le dérivé silicié de l'acide salicylique peut être appliquée par voie topique sur le visage, le cou, les cheveux, les muqueuses et les ongles ou toute autre zone cutanée du corps.

La solubilité des dérivés siliciés de l'acide salicylique selon l'invention dans les milieux organiques a été évaluée par comparaison avec le composé $C_1$ ci-dessus en mesurant la solubilité des composés $C_1$ à $C_3$ décrits ci-dessus, de l'acide 2-hydroxy-4-[2-méthyl-3-[1,3,3,3-tétraméthyl-1-[(triméthylsilyl)oxy]disiloxanyl]propyloxy]-benzoique (composé $C_4$) et de l'acide 2-hydroxy-3-[2-méthyl-3-[1,3,3,3-tétraméthyl-1-[(triméthylsilyl)oxy]-disiloxanyl]propyl]-benzoique (composé $C_5$), dans un mélange d'acide caprylique et de triglycéride d'acide caprique, commercialisé sous le nom de Miglyol 812 par la société Hūls.

Solubilité à température ambiante :

$C_1$ : <1,5%
$C_2$ : 2%
$C_3$ : 7%
$C_4$ : 6%
$C_5$ : >20%

Les dérivés selon l'invention étant plus solubles dans les huiles que les dérivés non siliciés de l'acide salicylique, ils sont plus facilement introduits dans des milieux hydrolipidiques et présentent une plus grande efficacité lorsqu'ils sont solubilisés dans ces milieux pour être ensuite appliqués sur la peau.

Des résultats identiques de desquamation et de solubilité peuvent être obtenus avec tous les produits selon l'invention.

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Dans les compositions selon l'invention, les produits selon les formules (**1**), (**2**) et (**3**) peuvent être utilisés en une quantité allant de 0,2 à 20 % en poids par rapport au poids total de la composition et en particulier en une quantité allant de 0,5 à 10 % et mieux de 0,5 à 5 % en poids par rapport au poids total de la composition.

Ces compositions constituent notamment des crèmes de protection, de traitement ou de soin de la peau ou des muqueuses pour le visage, pour les mains ou pour le corps, des laits corporels de protection ou de soin, des lotions, des gels ou des mousses pour le soin ou le traitement de la peau et des muqueuses ou pour le nettoyage de la peau.

Les compositions peuvent également consister en des préparations solides constituant des savons ou des pains de nettoyage.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la quantité d'huile peut aller jusqu'à plus de 90 % en poids du poids total de la composition.

De façon connue, la composition de l'invention peut contenir également des adjuvants habituels dans les domaines cosmétique et dermatologique, tels que les gélifiants hydrophiles ou lipophiles, les actifs hydrophiles ou lipophiles différents de ceux de l'invention, les conservateurs, les antioxydants, les solvants, les parfums, les agents séquestrants, les charges et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer le Polysorbate 60 et le stéarate de sorbitane vendus respectivement sous les dénominations commerciales Tween 60 et Span 60 par la Société ICI.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera, des antiseptiques.

Comme actifs lipophiles, on peut utiliser le tocophérol (vitamine E) et ses dérivés, les acides gras essentiels, les céramides, les huiles essentielles.

On peut, entre autre, associer les acides à des agents actifs destinés notamment à la prévention et/ou au traitement des affections cutanées. Parmi ces agents actifs, on peut citer à titre d'exemple :

- les agents modulant la différenciation et/ou la prolifération et/ou la pigmentation cutanée tels que la vitamine D et ses dérivés, les oestrogènes tels que l'oestradiol, l'acide kojique ou l'hydroquinone ;
- les agents anti-radicaux libres, tels que l'alpha-tocophérol ou ses esters, les superoxyde dismutases, certains chélatants de métaux ou l'acide ascorbique et ses esters.

On peut, par ailleurs, associer aux dérivé silicié de l'acide salicylique selon l'invention des antagonistes de substance P et/ou de CGRP (Calcitonin Gene Related Peptide ou peptide lié au gène de la calcitonine) tels que l'Iris Pallida et les sels de strontium, notamment les chlorures et les nitrates de strontium, ou des antagonistes de substance P et/ou de CGRP tels que ceux décrits dans les demandes de brevet français, l'une déposée au nom de la demanderesse sous le numéro 9500900, l'autre publiée sous le numéro FR-A-2719476. Une telle association permet de garantir une tolérance parfaite de ces compositions, même par des peaux très sensibles.

Le procédé de traitement cosmétique ou dermatologique de l'invention peuvent être mis en oeuvre notamment en appliquant les compositions hygiéniques, cosmétiques ou dermatologiques telles que définies ci-dessus, selon la technique d'utilisation habituelle de ces compositions. Par exemple : application de crèmes, de gels, de sérums, de pommades, de lotions, de laits sur la peau, le cuir chevelu, les ongles et/ou les muqueuses.

Les exemples suivants illustrent l'invention.

Les exemples ci-après illustrent l'invention sans en limiter la portée.

EXEMPLE 1:

Préparation du 2-hydroxy-4-(3-triméthylsilanyl-propyloxy)-benzoate de méthyle

A un mélange de 2,4-dihydroxy benzoate de méthyle (16,8g, 0,1 mole) et de carbonate de potassium (15,2g, 0.11mole) dans 100 ml de DMF, sous atmosphère d'azote, à 80°C, on ajoute goutte à goutte, en 10 minutes, du chloropropyl triméthyl silane (16,6 g, 0,11 mole). On chauffe le mélange à 90°C pendant 4 heures. On le refroidit et on le verse dans 200 ml d' eau. On extrait à l'éther diisopropylique. La phase organique est lavée à l'eau, séchée sur sulfate de sodium puis concentrée. Après chromatographie sur Silice de l'huile jaune obtenue (éluant : Heptane/$CH_2Cl_2$ 50:50), on récupère 28,2 g (Rendement : 57%) de 2-hydroxy-4-(3-triméthylsilanyl-propyloxy)-benzoate de méthyle sous la forme d'une poudre blanche

- Pf : 32-33°C.
- UV (Ethanol) $\lambda_{max}$ = 298 nm, $\varepsilon_{max}$ = 7325

| - Analyse élémentaire pour $C_{14}H_{22}O_4$ Si | | | |
|---|---|---|---|
| calculée | C 59,5 | H 7,8 | Si 10,0 |
| trouvée | C 59,3 | H 7,8 | Si 10,2 |

EXEMPLE 2 :

Préparation de l'acide 2-hydroxy-4-(3-triméthylsilanyl-propyloxy)-benzoique

On chauffe à 50°C le produit de l'exemple 1 (16 g, 0,057 mole) dans un mélange éthanol/eau 90:10 (100 ml) en présence de potasse (2 g) pendant 4 heures. On refroidit et on acidifie avec de l'acide chlorhydrique dilué. Le précipité formé est lavé à l'eau et séché sous vide. On obtient 15,2 g (Rendement : 93%) d'acide 2-hydroxy-4-(3-triméthylsilanyl-propyloxy)-benzoique sous la forme d'une poudre blanche.

- Pf: 187°C.
- UV (Ethanol) $\lambda_{max}$ = 295 nm, $\varepsilon_{max}$ = 6350

| - Analyse élémentaire pour $C_{13}H_{22}O_4$ Si | | | |
|---|---|---|---|
| théorique | C58,2 | H7,5 | Si10,4 |
| trouvée | C58,3 | H7,4 | Si10,1 |

EXEMPLE 3:

Préparation du 2-hydroxy-5-(3-triméthylsilanyl-propyloxyl-benzoate de méthyle

A un mélange de gentisate de méthyle (16,8g, 0,1 mole) et de carbonate de potassium (15,2g, 0,11mole) dans 80 ml de DMF, sous atmosphère d'azote, à 80°C, on ajoute goutte à goutte, en 20 minutes, du chloropropyl triméthyl silane (16,6g, 0,11mole). On chauffe le mélange à 90°C pendant 8 heures. On refroidit et on verse dans 200 ml d'eau. On extrait à l'éther diisopropylique. La phase organique est lavée à l'eau, séchée sur sulfate de sodium puis concentrée. Après chromatographie sur silice de l'huile jaune obtenue (éluant : Heptane/$CH_2Cl_2$ 90:10), on récupère 3,3 g d'une fraction propre de 2-hydroxy-5-(3-triméthylsilanyl-propyloxy)-benzoate de méthyle.

- UV (Ethanol) $\lambda_{max}$ = 335 nm, $\varepsilon_{max}$= 4500

| - Analyse élémentaire pour $C_{14}H_{22}O_4$ Si | | | |
|---|---|---|---|
| calculée | C 59.5 | H 7.8 | Si 9,9 |
| trouvée | C 59,5 | H 7,9 | Si 9,7 |

EXEMPLE 4 :

Préparation de l'acide 2-hydroxy-5-(3-triméthylsilanyl-propyloxy)-benzoique

On porte au reflux pendant 3 heures le produit de l'exemple 3 (3,3g, 0,012mole) dans un mélange éthanol/eau 50:50 (15 ml) en présence de potasse (2g). On refroidit et acidifie à pH 1 avec de l'acide chlorhydrique à 10%. Le précipité formé est lavé à l'eau et séché sous vide. Il est recristallisé dans un mélange méthanol/eau 50:50 (30 ml). On obtient 2g (Rendement : 64%) de l'acide 2-hydroxy-5-(3-triméthylsilanyl-propyloxy)-benzoique sous la forme d'une poudre blanche.

- Pf: 115-116°C.
- UV (Ethanol) $\lambda_{max}$ = 330 nm, $\varepsilon_{max}$ = 4335

| Analyse élémentaire pour $C_{13}$ $H_{20}$ $O_4$ Si | | | |
|---|---|---|---|
| théorique | C 58,2 | H 7,5 | Si 10,4 |
| trouvée | C 58,1 | H 7,5 | Si 10,2 |

EXEMPLE 5:

Préparation du 2-hydroxy-4-[2-méthyl-3-[1,3,3,3-tétraméthyl-1-[(triméthylsily)oxy]-disiloxanyl]propyloxy]benzoate de méthyle

a) Première étape : préparation du 2-hydroxy-4-(2-méthyl-allyloxy) benzoate de méthyle :

Dans un mélange de 2,4-dihydroxy benzoate de méthyle (24,5g, 0,145mole) et de carbonate de potassium (20,14g, 0,145mole) dans 90 ml de DMF porté à 75°C, on ajoute goutte à goutte en 30 minutes du chlorure de méthallyle (14,26ml, 0,145mole). On laisse 3 heures à 75°C. On refroidit et verse dans de l'eau glacée. On extrait au dichloro méthane et on lave la phase organique à l'eau, on la sèche sur sulfate de sodium, on évapore le solvant et on sèche sous vide.L'huile jaune pâle obtenue est purifiée sur silice (éluant : Heptane/$CH_2Cl_2$ 60:40). Les fractions de tête sont regroupées pour donner 13,4g du produit désiré sous forme d'une huile incolore.

b) Deuxième étape : préparation du 2-hydroxy-4-[2-méthyl-3-[1,3,3,3-tétraméthyl-1-[(triméthylsilyl)oxy]-disiloxanyl] propyloxy] benzoate de méthyle :

A une solution du dérivé précédent (13,4g, 0,06mole) et de catalyseur (complexe à 3-3.5% poids de Pt dans du cyclovinylméthylsiloxane de Hüls Petrarch PC085 : 100µl) dans 50ml de toluène sec porté à 100°C, on ajoute goutte à goutte en 10 minutes 14,12g d'heptaméthyltrisiloxane. On laisse à cette température pendant 30 minutes. On concentre le mélange réactionnel et on effectue ensuite une chromatographie sur silice sous pression (éluant : Heptane/

$CH_2Cl_2$ 85:15) On récupère alors 25 g (Rendement : 93%) de 2-hydroxy-4-[2-méthyl-3-[1,3,3,3-tétraméthyl-1-[(triméthylsilyl)oxy]disiloxanyl] propyloxy] benzoate de méthyle sous la forme d'une huile jaune très pâle.

- -UV (Ethanol) $\lambda_{max}$ = 298 nm, $\varepsilon_{max}$ = 7340

| - Analyse élémentaire pour $C_{19}H_{36}O_6Si_3$ | | | |
|---|---|---|---|
| théorique | C 51,3 | H 8,2 | Si 18,9 |
| trouvée | C 51,6 | H 8,2 | Si 19,0 |

EXEMPLE 6 : Préparation du 2-hydroxy-3-[2-méthyl-3-[1,3,3,3-tétraméthyl-1-[(triméthysilyl)oxy]disiloxanyl]propyl] benzoate de méthyle

a) Première étape : Préparation du 2-(2-méthyl-allyloxy)de méthyle :

Dans un mélange de 2-hydroxy benzoate de méthyle (60g, 0,394mole) et de carbonate de potassium (52,6g, 0,398mole) dans 240ml de DMF porté à 70°C, on ajoute goutte à goutte en 20 minutes du chlorure de méthallyle (39ml, 0,398mole). On laisse 5 heures à 70°C. On refroidit et enlève les sels inorganiques par filtration. Le DMF est évaporé sous vide. On obtient le dérivé attendu sous forme d'une huile brun pâle (74,6g, Rendement : 92%).

b) Deuxième étape : préparation du 2-hydroxy-3-(2-méthyl-allyl)benzoate de méthyle :

La totalité du dérivé précédent est chauffée à 190°C sous azote pendant 10 heures. Ce brut de réaction est refroidit et soumis à une distillation sous vide. On recueille la fraction qui distille à 68-70°C sous un vide de 0.2 mmHg et qui correspond au 2-hydroxy-3-(2-méthyl-allyl) benzoate de méthyle (63,4g, Rendement : 70%).

c) Troisième étape : préparation du 2-hydroxy-3-[2-méthyl-3-[1,3,3,3-tétraméthyl-1-[(triméthylsilyl)oxy]disiloxanyl]propyl]benzoate de méthyle :

A une solution de 25g (0,121 mole) du dérivé précédent et de 100µl de catalyseur (complexe à 3-3,5% en poids de platine cyclovinylméthylsiloxane commercialisé par la société Huls petrarch sous la référence PC085) dans 80ml de toluène à une température de 100°C, on ajoute au goutte à goutte en 2h 28,4g d'heptaméthylsiloxane. On agite à cette température pendant 1h, puis on concentre le milieu sous vide et on purifie le produit par chromatographie sur colonne de silice (éluant: Heptane/$CH_2Cl_2$ 80/20). On récupère ainsi 38g (rendement : 73%) de 2-hydroxy-3-[2-méthyl-3-[1,3,3,3-tétraméthyl-1-[(triméthylsilyl)oxy]-disiloxanyl]propyl] benzoate de méthyle sous la forme d'une huile jaune pâle.

- -UV (Ethanol) $\lambda_{max}$ = 313 nm, $\varepsilon_{max}$ = 4615

| - Analyse élémentaire pour $C_{19}H_{36}O_5Si_3$ | | | |
|---|---|---|---|
| théorique | C 51,2 | H 8,5 | Si 19,65 |
| trouvée | C 53,2 | H 8,5 | Si 19,4 |

EXEMPLE 7 :

Préparation du 2-hydroxy-4-[2-méthyl-3-[1,3,3,3-tétraméthyl-1-[(triméthylsilyl)oxyl]-di-siloxanyl]propyloxy]-benzoate de benzyle.

a) Première étape : préparation du 2,4-dihydroxy benzoate de benzyle:

A 76,8 g de sel de potassium de l'acide résorcylique (0,4 mole) dans 500 ml d'éthanol à 95%, on ajoute 50 ml de chlorure de benzyle (0,42 mole). On laisse au reflux pendant 2 heures, on concentre à la moitié, puis on ajoute 500 ml d'eau. L'huile qui décante est lavée avec une solution de bicarbonate de sodium, puis avec de l'heptane. Le solide résultant est séché sous vide et utilisé tel quel dans l'étape suivante.

b) Deuxième étape : préparation du 2-hydroxy-4-(2-méthyl-allyloxy)-benzoate de benzyle:

Dans un mélange de 17 g de 2,4-dihydroxy benzoate de benzyle (0,07 mole) et de 10 g de carbonate de potassium (0,077 mole) dans 60 ml de DMF porté à 70°C, on ajoute goutte à goutte en 1 heure 6,81 ml de chlorure de méthallyle (0,07 mole). On laisse 2 heures à 70°C. On refroidit vers 40°C et on filtre les sels. Les sels sont lavés au DMF. Les jus sont rassemblés et le solvant évaporé. On obtient une huile brûne que l'on purifie sur silice (éluant : Heptane/ $CH_2Cl_2$ 70:30). Les fractions de tête sont regroupées pour donner 12 g (Rendement : 57%) de 2-hydroxy-4-(2-méthyl-allyloxy)-benzoate de benzyle sous forme d'une huile incolore.

c) Troisième étape : préparation du 2-hydroxy-4-[2-méthyl-3-[1,3,3,3-tétraméthyl-1- [(triméthylsilyl)oxy]disiloxanyl]propyloxy]-benzoate de benzyle:

A une solution de 12 g du dérivé précédent (0,04 mole) et de 100µl de catalyseur (complexe à 3-3.5% poids de Pt dans du cyclovinylméthylsiloxane de Hüls Petrarch PC085) dans 40 ml de toluène sec porté à 100°C, on ajoute goutte à goutte en 30 minutes 9,45 g d'heptaméthyltrisiloxane. On laisse à cette température pendant 6 heures. On concentre le mélange réactionnel et on effectue ensuite une chromatographie sur silice sous pression (éluant : Heptane/$CH_2Cl_2$ 80:20). On récupère alors 17,2 g (Rendement : 82%) de 2-hydroxy-4-(2-méthyl-3-[1,3,3,3-tétraméthyl-1 [(triméthylsilyl)oxy]disiloxanyl]propyloxy]-benzoate de benzyle sous la forme d'une huile incolore.

- UV (Ethanol) $\lambda_{max}$ = 298 nm, $\varepsilon_{max}$ = 8 130

| - Analyse élémentaire pour $C_{25}H_{40}O_6Si_3$ | | | |
|---|---|---|---|
| théorique | C 57.6 | H 7.7 | Si 16.2 |
| trouvée | C 57.3 | H 7.9 | Si 16.4 |

EXEMPLE 8 :

Préparation de l'acide 2-hydroxy-4-[2-méthyl-3-[1,3,3,3-tétraméthyl-1-[(triméthylsilyl)oxy]disiloxanyl]propyloxyl]-benzoique

On porte au reflux un mélange de 12 g de 2-hydroxy-4-(2-méthyl-3-[1,3,3,3-tétraméthyl-1-[(triméthylsilyl)oxy]disiloxanyl]propyloxy]-benzoate de benzyle (0,023 mole), d'éthanol (40 ml), de cyclohéxène (12 ml) et de Palladium à 10% sur charbon (1,2 g) pendant 1 heure. On refroidit et on rince avec de l'éthanol. Les jus sont concentrés et le produit résultant est recristallisé dans un mélange eau/éthanol 50:50 pour donner l'acide 2-hydroxy-4-[2-méthyl-3-[1,3,3,3-tétraméthyl-1-[(triméthylsilyl)oxy]-disiloxanyl] propyloxy]-benzoique sous la forme d'une poudre blanche.

- Pf : 117-118 °C.
- UV (Ethanol) $\lambda_{max}$ = 294 nm, $\varepsilon_{max}$ = 6 650

| - Analyse élémentaire pour $C_{18}H_{34}O_6Si_3$ | | | |
|---|---|---|---|
| théorique | C 50.1 | H 8.0 | Si 19.6 |
| trouvée | C 49.9 | H 8.0 | Si 19.9 |

EXEMPLE 9 :

Préparation de l'acide 2-hydroxy-3-[2-méthyl-3-[1,3,3,3-tétraméthyl-1-[(triméthylsilyl) oxy]disiloxanyl]propyl]-benzoique

a) Première étape : préparation du 2-(2-méthyl-allyloxy)-benzoate de benzyle :

Dans un mélange de 2-hydroxy benzoate de benzyle (114,1 g, 0,5 mole) et de carbonate de potassium (66,8g, 0,505 mole) dans 300 ml de DMF porté à 70°C, on ajoute goutte à goutte en 20 minutes du chlorure de méthallyle (51,4 ml, 0,505 mole). On laisse 3 heures à 70°C. On refroidit vers 40°C et on filtre les sels. Les sels sont lavés au DMF. Les jus sont rassemblés et le solvant évaporé. On obtient 140 g (Rendement 100 %) d'une huile brûne de 2-(2-méthyl-allyloxy)-benzoate de benzyle que l'on utilise tel quel pour l'étape suivante.

b) Deuxième étape : préparation du 2-hydroxy-3-(2-méthyl-allyl)-benzoate de benzyle :

Le dérivé précédent est chauffé pendant 8 heures à 190 °C. Après refroidissement, le mélange réactionnel est soumis à une distillation fractionnée. On récupère la fraction qui passe à 85-90 °C sous un vide de 0,4 mmHg. On obtient ainsi 105 g (Rendement : 90 %) de 2-hydroxy-3-(2-méthyl-3-allyl)-benzoate de benzyle sous la forme d'une huile incolore.

c) Troisième étape : préparation du 2-hydroxy-3-[2-méthyl-3-[1,3,3,3-tétraméthyl-1-[(triméthylsilyl)oxy]disiloxanyl]propyl]-benzoate de benzyle:

A une solution du dérivé précédent (59 g, 0,209 mole) et de catalyseur (complexe à 3-3.5% poids de Pt dans du cyclovinylméthylsiloxane de Hüls Petrarch PC085 : 200 µl) dans 100 ml de toluène sec porté à 80°C, on ajoute goutte à goutte en 4 heures 48,83 g d'heptaméthyltrisiloxane. On laisse à cette température pendant 1 heure. On concentre le mélange réactionnel et on obtient 104 g d'une huile brute jaune pâle. Cette huile (9 g) est purifiée par chromatographie sur silice sous pression (éluant : Heptane/$CH_2Cl_2$ 70:30). On récupère alors 7,8 g de 2-hydroxy-3-(2-méthyl-3-[1,3,3,3-tétraméthyl-1-[(triméthylsilyl)oxy]disiloxanyl]propyl]-benzoate de benzyle sous la forme d'une huile incolore.

- -UV (Ethanol) $\lambda_{max}$ = 314 nm, $\varepsilon_{max}$ = 5 500

| - Analyse élémentaire pour $C_{25}H_{40}O_5Si_3$ | | | |
|---|---|---|---|
| théorique | C59.5 | H8.0 | Si16.7 |
| trouvée | C59.5 | H7.9 | Si17.0 |

EXEMPLE 10:

Préparation de l'acide 2-hydroxy-3-[2-méthyl-3-[1,3,3,3-tétraméthyl-1-[(triméthylsilyl) oxy]disiloxanyl]propyl]-benzoique

On porte au reflux un mélange de l'huile brute précédente de 2-hydroxy-3-(2-méthyl-3-[1,3,3,3-tétraméthyl-1-[(triméthylsilyl)oxy]disiloxanyl]propyl]-benzoate de benzyle (11,61 g, 0,023 mole), d'éthanol (40 ml), de cyclohexène (12 ml) et de Palladium à 10% sur charbon (1,2 g) pendant 1 heure. On refroidit et on rince avec de l'éthanol. Les jus sont concentrés et l'huile jaune pâle obtenue est purifiée par chromatographie sur silice sous pression (éluant: $CH_2Cl_2$ / Acetate d'éthyle / Acide acétique 80:20:0,2). pour donner 7,5 g (Rendement : 78%) d'acide 2-hydroxy-3-[2-méthyl-3-[1,3,3,3-tétraméthyl-1-[(triméthylsilyl)oxy]disiloxanyl]propyl]-benzoique sous la forme d'une huile incolore.

- UV (Ethanol) $\lambda_{max}$ = 310 nm, $\varepsilon_{max}$ = 4 550

| - Analyse élémentaire pour $C_{18}H_{34}O_5Si_3$ | | | |
|---|---|---|---|
| théorique | C52.1 | H8.3 | Si20.3 |
| trouvée | C52.2 | H8.2 | Si20.4 |

EXEMPLE 11:

Préparation de l'acide 2-hydroxy-5-[3-[1,3,3,3-tétraméthyl-1-(triméthylsilyl)oxy] disiloxanyl]propylamido]-benzoique.

A une solution d' acide 4-hydroxy-isophthalique (5 g, 0,0275 mole) dans le DMF (25 ml) à 20°C, on ajoute en 10 minutes du chlorure de thionyle (2,2 ml, 0,03 mole) et laisse réagir pendant 1 heure.

Cette solution jaune est versée en 1 heure à un mélange de triéthylamine (6,1 g, 0,06 mole) et d' heptaméthyl aminopropyl trisiloxane (8,4 g, 0,03 mole). Le mélange qui devient hétérogène est laissé sous agitation pendant 6 heures à 20°C.

Ce mélange réactionnel est versé dans l'eau. La pâte gommeuse obtenue est triturée avec de l'eau, puis avec du dichloro méthane. Le précipité obtenu est recristallisé dans un mélange éthanol eau pour donner 2,5 g (Rendement : 20 %) d'acide 2-hydroxy-5-[3-[1,3,3,3-tétraméthyl-1-[(triméthylsilyl)oxy]disiloxanyl]propylamido]-benzoique sous la forme d'une poudre blanche.

- Pf: 222-224 °C
- UV (Ethanol) $\lambda_{max}$ = 308 nm, $\varepsilon_{max}$ = 4 100

| - Analyse élémentaire pour $C_{18}H_{33}O_6Si_3$ | | | | |
|---|---|---|---|---|
| théorique | C48.7 | H7.5 | N3.1 | Si19.0 |
| trouvée | C48.9 | H7.4 | N2.9 | Si19.0 |

Exemple de composition :

Cet exemple illustre l'invention. Les proportions indiquées sont des pourcentages en poids.

Composition 1 : Emulsion H/E

*Phase A* :

- Acide 2-hydroxy-4-(3-triméthylsilanyl-propyloxy)-benzoique 2,5
- Huile d'amande douce 14,5
- Huile de karité 7,0
- PPG-3 myristyl éther (EMCOL 249-3K) 5,0
- Conservateur (propylparabène) 0,1
- Polysorbate 60 (TWEEN 60) 2,5
- Stéarate de sorbitane (SPAN 60) 2,5

*Phase B* :

- Cyclométhicone 4,0
- Gomme de xanthane 0,2
- Polymère carboxyvinylique 0,5

*Phase C* :

- Triéthanolamine (neutralisant) 0,5
- Eau 2,0

*Phase D* :

- Conservateur (méthylparabène) 0,2
- Glycérine 5,0
- Eau qsp 100

Mode opératoire :

On fait fondre les constituants de la phase A à 85° C, puis on refroidit la phase A à 70° C et on y introduit les phases B puis C et D sous agitation. On refroidit jusqu'à la température ambiante. On obtient une crème de jour qui provoque la desquamation de la peau et confère ainsi à celle-ci un aspect plus lisse et plus jeune qu'avant le traitement.

**Revendications**

1. Produit répondant à l'une des formules (1) à (3) suivantes :

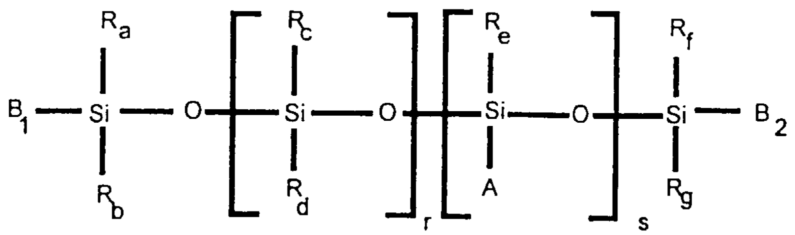

(1)

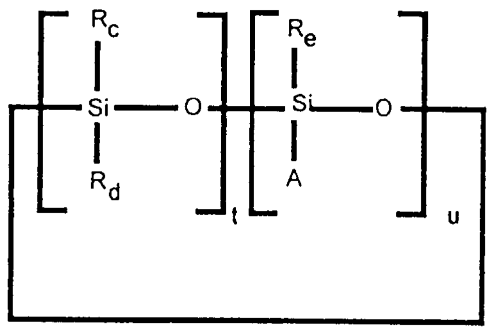

(2)

$$A\text{-}SiR'_1R'_2R'_3 \qquad (3)$$

dans lesquelles :

- Ra à Rg, identiques ou différents, sont choisis parmi le groupe constitué des radicaux alcoyles en $C_1$-$C_{10}$ et alcényles en $C_2C_{10}$, linéaires ou ramifiés, saturés ou insaturés, le radical phényle et le radical trifluoro-3,3,3 propyle, au moins 80% en nombre des radicaux Ra à Rg étant le radical méthyle.
- A désigne un radical monovalent lié directement à un atome de silicium et répondant à la formule (4) :

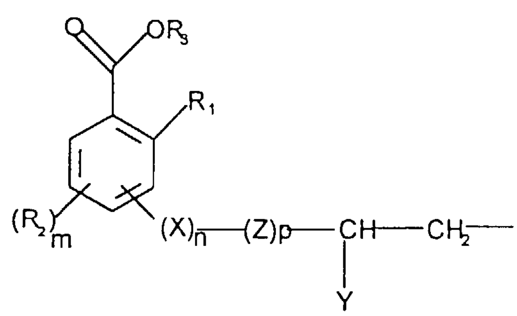

**(4)**

dans laquelle :

$R_1$ représente OH, un radical alcoxyle en $C_1$-$C_4$, linéaire ou ramifié, saturé ou insaturé, ou une fonction acyloxy de formule $O(C{=}O)R_4$, dans laquelle $R_4$ représente un radical alcoyle en $C_1$-$C_8$ ou alcényle en $C_2$-$C_8$, linéaire ou ramifié,
$R_2$, identiques ou différents, représentent un radical OH, alcoyle en $C_1$-$C_8$ ou alcényle en $C_2$-$C_8$, linéaire ou ramifié, alcoxyle en $C_1$-$C_8$, linéaire ou ramifié, deux $R_2$ adjacents pouvant former ensemble un groupement alcanedioxy- dans lequel la chaîne alcane est constituée de 1 ou 2 atomes de carbone,
$R_3$ représente un radical choisi parmi: un atome d'hydrogène, un cation pharmaceutiquement acceptable, un radical alcoyle en $C_1$-$C_6$ ou alcényle en $C_2$-$C_6$, linéaire ou ramifié et un radical benzyle, éventuellement

substitué par un groupement choisi parmi les radicaux : alcoyle en $C_1$-$C_6$ ou alcényle en $C_2$-$C_6$, linéaire ou ramifié, hydroxy, amino, alcoxy en $C_1$-$C_6$ ou alcényloxy en $C_2$-$C_6$, linéaire ou ramifié, halogéno, acide carboxylique, carboxylate d'alcoyle en $C_1$-$C_6$ ou carboxylate d'alcényle en $C_2$-$C_6$, linéaire ou ramifié, lorsque $R_1$ est différent de OH, $R_3$ est H ou un cation pharmaceutiquement acceptable,
m est choisi parmi les entiers 0, 1 ou 2,
p est choisi parmi les entiers 0 et 1,
X représente O, NH , C=O, , NH(C=O)NH, NH(C=O) ou (C=O)NH,
n est choisi parmi les entiers 0 ou 1,
Z est un radical alcane di-yle en $C_1$-$C_6$ linéaire ou ramifié, saturé ou insaturé, éventuellement subtitué par un radical hydroxyle ou alcoxyle en $C_2$-$C_8$, linéaire ou ramifié, saturé ou insaturé,
Y représente un atome d'hydrogène, un radical hydroxyle ou un radical alcoxyle en $C_2$-$C_8$, linéaire ou ramifié, saturé ou insaturé,

- $B_1$ et $B_2$, identiques ou différents, sont choisis parmi les radicaux Ra à Rg et A définis ci-dessus,

- $R'_1$, $R'_2$, $R'_3$, identiques ou différents, sont choisis parmi les radicaux alcoyles en $C_1$-$C_8$ et alcényles en $C_2$-$C_8$, linéaires ou ramifiés, saturés ou insaturés, le radical phényle, et les radicaux alcoxyles en $C_1$-$C_4$, linéaires ou ramifiés, saturés ou insaturés,

- r est un nombre entier allant de 0 à 50,

- s est un nombre entier allant de 0 à 20, étant entendu que si s est nul, alors au moins l'un des radicaux $B_1$ et $B_2$ désigne le radical A,

- u est un nombre entier allant de 1 à 6, t est un nombre entier allant de 0 à 10, étant entendu que t+u est supérieur ou égal à 3,

2. Produit selon la revendication 1, caractérisé en ce que dans la formule (4), le chaînon $-(X)_n$-Z- est accroché sur le noyau aromatique en position 3, 4 ou 5.

3. Produit selon l'une quelconque des revendications précédentes, caractérisé en ce que les radicaux Ra à Rg sont choisis parmi les radicaux méthyle, éthyle, propyle, n-butyle, n-octyle et éthyl-2 hexyle.

4. Produit selon la revendication précédente, caractérisé en ce que les radicaux Ra à Rg représentent le méthyle.

5. Produit selon l'une quelconque des revendications précédentes, caractérisé en ce que les radicaux $R'_1$, $R'_2$, $R'_3$ sont choisis parmi les radicaux méthyle, éthyle, propyle, n-butyle, n-octyle et éthyl-2 hexyle.

6. Produit selon la revendication précédente, caractérisé en ce que les radicaux $R'_1$, $R'_2$, $R'_3$ représentent le méthyle.

7. Produit selon l'une quelconque des revendications précédentes, caractérisé en ce que les radicaux alkyles $B_1$ et $B_2$ sont choisis parmi les radicaux méthyle, éthyle, propyle, n-butyle, n-octyle et éthyl-2 hexyle.

8. Produit selon la revendication précédente, caractérisé en ce que les radicaux $B_1$ et $B_2$ représentent tous deux le méthyle.

9. Produit selon l'une quelconque des revendications précédentes, caractérisé en ce que r est un nombre entier allant de 0 à 3.

10. Produit selon l'une quelconque des revendications précédentes, caractérisé en ce que s est un nombre entier allant de 0 à 3.

11. Produit selon l'une quelconque des revendications précédentes, caractérisé en ce que $R_1$ est OH.

12. Produit selon l'une quelconque des revendications précédentes, caractérisé en ce que $R_3$ est l'atome d'hydrogène.

13. Produit selon l'une quelconque des revendications précédentes, caractérisé en ce que m = 0.

**14.** Produit selon l'une quelconque des revendications précédentes, caractérisé en ce que n=0 ou bien n=1 et X représente O.

**15.** Produit selon l'une quelconque des revendications précédentes, caractérisé en ce qu'il est choisi parmi :

- le 2-hydroxy-4-(3-triméthylsilanyl-propyloxy)-benzoate de méthyle,
- l'acide 2-hydroxy-4-(3-triméthylsilanyl-propyloxy)-benzoique,
- le 2-hydroxy-5-(3-triméthylsilanyl-propyloxy)-benzoate de méthyle,
- l'acide 2-hydroxy-5-(3-triméthylsilanyl-propyloxy)-benzoique,
- le 2-hydroxy-4-[2-méthyl-3-[1,3,3,3-tétraméthyl-1-[(triméthylsilyl)oxy]-disiloxanyl]-propyloxy]benzoate de méthyle,
- le 2-hydroxy-3-[2-méthyl-3-[1,3,3,3-tétraméthyl-1-[(triméthylsilyl)oxy]-disiloxanyl]-propyl] benzoate de méthyle,
- l'acide 2-hydroxy-4-[2-méthyl-3-[1,3,3,3-tétraméthyl-1-[(triméthylsilyl)oxy]-disiloxanyl]propyl]-benzoique,
- l'acide 2-hydroxy-3-[2-méthyl-3-[1,3,3,3-tétraméthyl-1-[(triméthylsilyl)oxy]-disiloxanyl]propyl]-benzoique,
- l'acide 2-hydroxy-5-[3-[1,3,3,3-tétraméthyl-1-[(triméthylsilyl)oxy]disiloxanyl]-propylamido]-benzoique.

**16.** Utilisation d'un produit selon l'une quelconque des revendications précédentes dans ou pour la fabrication d'une composition cosmétique ou dermatologique pour favoriser la desquamation de la peau humaine et/ou stimuler le renouvellement épidermique.

**17.** Utilisation d'un produit selon l'une quelconque des revendications 1 à 15 pour traiter le vieillissement intrinsèque et extrinsèque de la peau humaine.

**18.** Utilisation d'un produit selon l'une quelconque des revendications 1 à 15 dans ou pour la fabrication d'une composition cosmétique ou dermatologique comme agent antivieillissement, pour lutter contre les rides et/ou les ridules et/ou les taches actiniques et/ou les dyschromies cutanées et/ou les dermites et/ou les cicatrices de la peau humaine.

**19.** Utilisation d'un produit selon l'une quelconque des revendications 1 à 15 dans ou pour la fabrication d'une composition cosmétique ou dermatologique anti-bactérienne.

**20.** Utilisation d'un produit selon l'une quelconque des revendications 1 à 15 dans ou pour la fabrication d'une composition cosmétique ou dermatologique anti-solaire

**21.** Utilisation d'un produit selon l'une quelconque des revendications 1 à 15 dans ou pour la fabrication d'une composition cosmétique ou dermatologique de protection de la peau contre les radicaux libres.

**22.** Procédé de traitement non thérapeutique de la peau destiné à la desquamation de la peau consistant à appliquer sur la peau humaine une composition contenant une quantité efficace d'au moins un produit selon l'une quelconque des revendications 1 à 15 dans un milieu cosmétiquement et/ou dermatologiquement acceptable.

**23.** Procédé de traitement non thérapeutique du vieillissement de la peau humaine, consistant à appliquer sur cette peau une composition contenant une quantité efficace d'au moins un produit selon l'une quelconque des revendications 1 à 15 dans un milieu cosmétiquement et/ou dermatologiquement acceptable.

**24.** Composition cosmétique et/ou dermatologique, caractérisée en ce qu'elle comprend dans un support cosmétiquement ou dermatologiquement acceptable, au moins un produit selon l'une quelconque des revendications 1 à 15.

**25.** Composition selon la revendication 24, caractérisée en ce qu'elle est sous la forme d'une crème de protection, de traitement ou de soin de la peau ou des muqueuses pour le visage, pour les mains ou pour le corps, d'un lait corporel de protection ou de soin, d'une lotion, d'un gel ou d'une mousse pour le soin ou le traitement de la peau et des muqueuses ou pour le nettoyage de la peau, d'un savon ou d'un pain de nettoyage.

**26.** Composition selon la revendication 24 ou 25, caractérisée en ce qu'elle comprend en outre au moins un produit choisi parmi les antagonistes de substance P et/ou de CGRP (Calcitonin Gene Related Peptide ou peptide lié au gène de la calcitonine).

**27.** Composition selon l'une quelconque des revendications 24 à 26, caractérisée en ce qu'elle comprend 0,2 à 20 % en poids par rapport au poids total de la composition d'un produit selon l'une des revendications 1 à 15.

**Patentansprüche**

**1.** Verbindung nach einer der folgenden Formeln (1) bis (3)

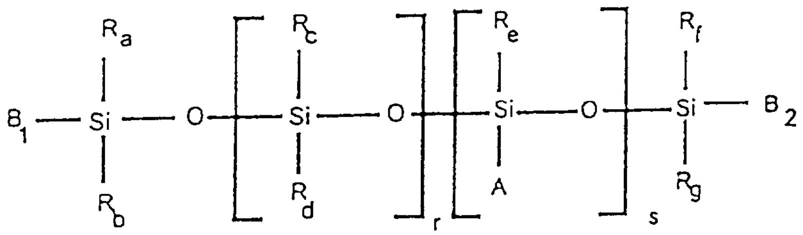

(1)

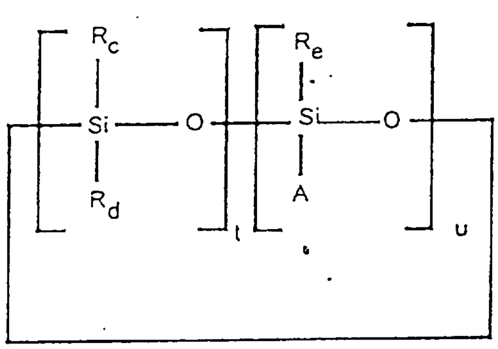

(2)

$$A\text{-}SiR'_1R'_2R'_3 \qquad (3)$$

in denen bedeuten:

- Ra bis Rg, die gleich oder verschieden sind, Gruppen, die unter geradkettigen oder verzweigten, gesättigten oder ungesättigten $C_{1-10}$-Alkylguppen und $C_{2-10}$-Alkenylgruppen, Phenyl und 3,3,3-Trifluorpropyl ausgewählt sind, wobei mindestens 80 % der Gruppen Ra bis Rg, bezogen auf die Anzahl der Gruppen, Methylgruppen sind,

- A eine einwertige Gruppe, die unmittelbar mit einem Siliciumatom verbunden ist und die Formel (4)

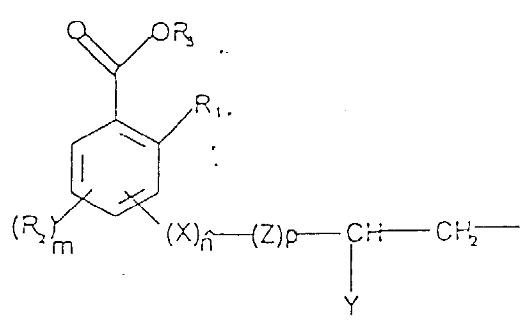

(4)

aufweist, in der bedeuten:

- $R_1$ OH, eine geradkettige oder verzweigte, gesättigte oder ungesättigte $C_{1-4}$-Alkoxygruppe oder eine Acyloxygruppe der Formel $O(C=O)R_4$, worin $R_4$ eine geradkettige oder verzweigte $C_{1-8}$-Alkylgruppe oder $C_{2-8}$-Alkenylgruppe darstellt,
- $R_2$, die gleich oder verschieden sind, eine OH-Gruppe, eine geradkettige oder verzweigte $C_{1-8}$-Alkyl- oder $C_{2-8}$-Alkenylgruppe, eine geradkettige oder verzweigte $C_{1-8}$-Alkoxygruppe, wobei zwei benachbarte Gruppen $R_2$ zusammen eine Alkandioxygruppe bilden können, deren Alkankette aus 1 bis 2 Kohlenstoffatomen besteht,
- $R_3$ eine Gruppe, die unter Wasserstoff, pharmazeutisch akzeptablen Kationen, geradkettigem oder verzweigtem $C_{1-6}$-Alkyl oder $C_{2-6}$-Alkenyl und Benzyl ausgewählt ist, die gegebenenfalls mit einer Gruppe substituiert ist, die ausgewählt ist unter geradkettigem oder verzweigtem $C_{1-6}$-Alkyl oder $C_{2-6}$-Alkenyl, Hydroxy, Amino, geradkettigem oder verzweigtem $C_{1-6}$-Alkoxy oder $C_{2-6}$-Alkenyloxy, Halogenatomen, der Carbosäuregruppe, Carboxylaten mit geradkettigen oder verzweigten $C_{1-6}$-Alkyl oder $C_{2-6}$-Alkenyl,
  wobei $R_3$ Wasserstoff oder ein pharmazeutisch akzeptables Kation ist, wenn $R_1$ keine OH-Gruppe ist, m eine Zahl, die unter den ganzen Zahlen 0, 1 und 2 ausgewählt ist,

  p eine Zahl, die unter den ganzen Zahlen 0 und 1 ausgewählt ist,
  X Sauerstoff, NH, C=O, NH(C=O)NH, NH(C=O) oder (C=O)NH,
  n eine Zahl, die unter den ganzen Zahlen 0 und 1 ausgewählt ist,
  Z eine geradkettige oder verzweigte, gesättigte oder ungesättigte, gegebenenfalls mit einer Hydroxygruppe oder einer geradkettigen oder verzweigten, gesättigten oder ungesättigten $C_{2-8}$-Alkoxygruppe substituierte $C_{1-6}$-Alkandiylgruppe,
  Y ein Wasserstoffatom, eine Hydroxygruppe oder eine geradkettige oder verzweigte, gesättigte oder ungesättigte $C_{2-8}$-Alkoxygruppe,

- $B_1$ und $B_2$, die gleich oder verschieden sind, Gruppen, die unter den weiter oben definierten Gruppen Ra bis Rg und A ausgewählt sind,
- $R_1'$, $R_2'$, $R_3'$, die gleich oder verschieden sind, Gruppen, die unter geradkettigem oder verzweigtem, gesättigtem oder ungesättigtem $C_{1-8}$-Alkyl und $C_{2-8}$-Alkenyl, Phenyl, geradkettigen oder verzweigten, gesättigten oder ungesättigten $C_{1-4}$-Alkoxygruppen ausgewählt sind,
- r eine ganze Zahl im Bereich von 0 bis 50,
- s eine ganze Zahl im Bereich von 0 bis 20 mit der Maßgabe, daß, wenn s Null ist, mindestens eine der beiden Gruppen $B_1$ und $B_2$ die Gruppe A bedeutet,
- u eine ganze Zahl im Bereich von 1 bis 6, t eine ganze Zahl im Bereich von 0 bis 10 mit der Maßgabe, daß t+u größer als oder gleich 3 ist.

**2.** Verbindung nach Anspruch 1, dadurch gekennzeichnet, daß in Formel (4) das Kettenglied $-(X)_n-Z-$ in 3-, 4- oder 5-Stellung an den aromatischen Kern gebunden ist.

**3.** Verbindung nach einem der vorhergehenden Ansprüche, dadurch gekennzeichnet, daß die Reste Ra bis Rg unter Methyl, Ethyl, Propyl, n-Butyl, n-Octyl und 2-Ethylhexyl ausgewählt sind.

**4.** Verbindung nach dem vorhergehenden Anspruch, dadurch gekennzeichnet, daß die Reste Ra bis Rg Methylreste sind.

**5.** Verbindung nach einem der vorhergehenden Ansprüche, dadurch gekennzeichnet, daß die Reste $R'_1$, $R'_2$, $R'_3$ unter Methyl, Ethyl, Propyl, n-Butyl, n-Octyl und 2-Ethylhexyl ausgewählt sind.

**6.** Verbindung nach dem vorhergehenden Anspruch, dadurch gekennzeichnet, daß die Reste $R'_1$, $R'_2$, $R'_3$ Methylreste sind.

**7.** Verbindung nach einem der vorhergehenden Ansprüche, dadurch gekennzeichnet, daß die Alkylreste $B_1$ und $B_2$ unter Methyl, Ethyl, Propyl, n-Butyl, n-Octyl und 2-Ethylhexyl ausgewählt sind.

**8.** Verbindung nach dem vorhergehenden Anspruch, dadurch gekennzeichnet, daß sowohl der Rest $B_1$ als auch der Rest $B_2$ ein Methylrest ist.

**9.** Verbindung nach einem der vorhergehenden Ansprüche, dadurch gekennzeichnet, daß r eine ganze Zahl im Bereich von 0 bis 3 ist.

**10.** Verbindung nach einem der vorhergehenden Ansprüche, dadurch gekennzeichnet, daß s eine ganze Zahl von 0 bis 3 ist.

**11.** Verbindung nach einem der vorhergehenden Ansprüche, dadurch gekennzeichnet, daß $R_1$ OH ist.

**12.** Verbindung nach einem der vorhergehenden Ansprüche, dadurch gekennzeichnet, daß $R_3$ das Wasserstoffatom ist.

**13.** Verbindung nach einem der vorhergehenden Ansprüche, dadurch gekennzeichnet, daß m = 0 ist.

**14.** Verbindung nach einem der vorhergehenden Ansprüche, dadurch gekennzeichnet, daß n gleich Null oder 1 ist und X Null ist.

**15.** Verbindung nach einem der vorhergehenden Ansprüche, dadurch gekennzeichnet, daß sie ausgewählt ist unter:

- 2-Hydroxy-4-(3-trimethylsilanylpropyloxy)benzoesäuremethylester,
- 2-Hydroxy-4-(3-trimethylsilanylpropyloxy)benzoesäure,
- 2-Hydroxy-5-(3-trimethylsilanylpropyloxy)-benzoesäuremethylester,
- 2-Hydroxy-5-(3-trimethylsilanylpropyloxy)-benzoesäure,
- 2-Hydroxy-4-[2-methyl-3-[1,3,3,3-tetramethyl-1-[(trimethylsilyl)oxy]-disiloxanyl]-propyloxy]-benzoe-säuremethylester,
- 2-Hydroxy-3-[2-methyl-3-[1,3,3,3-tetramethyl-1-[(trimethylsilyl)oxy]-disiloxanyl]-propyl]-benzoe-säuremethylester,
- 2-Hydroxy-4-[2-methyl-3-[1,3,3,3-tetramethyl-1-[(trimethylsilyl)oxy]-disiloxanyl]-propyl]-benzoe-säure,
- 2-Hydroxy-3-[2-methyl-3-[1,3,3,3-tetramethyl-1-[(trimethylsilyl)oxy]-disiloxanyl]propyl]-benzoe-säure,
- 2-Hydroxy-5-[3-[1,3,3,3-tetramethyl-1-[(trimethyl-silyl]oxy]-disiloxanyl]-propylamido]-benzoesäure.

**16.** Verwendung einer Verbindung nach einem der vorhergehenden Ansprüche in einer oder für die Herstellung einer kosmetischen oder dermatologischen Zusammensetzung zur Förderung der Abschuppung der menschlichen Haut und/oder zur Anregung der Erneuerung der Epidermis.

**17.** Verwendung einer Verbindung nach einem der Ansprüche 1 bis 15 für die Behandlung der inneren und äußeren Alterung der menschlichen Haut.

**18.** Verwendung einer Verbindung nach einem der Ansprüche 1 bis 15 in einer oder für die Herstellung einer kosmetischen oder dermatologischen Zusammensetzung als Mittel gegen die Alterung, zur Bekämpfung von Falten und/oder Fältchen und/oder von lichtbedingten Flecken und/oder von Dyschromien der Haut und/oder von Hautentzündungen und/oder von Narben der menschlichen Haut.

**19.** Verwendung einer Verbindung nach einem der Ansprüche 1 bis 15 in einer oder für die Herstellung einer antibakteriellen kosmetischen oder dermatologischen Zusammensetzung.

**20.** Verwendung einer Verbindung nach einem der Ansprüche 1 bis 15 in einer oder für die Herstellung einer kosmetischen oder dermatologischen Lichtschutzzusammensetzung.

**21.** Verwendung einer Verbindung nach einem der Ansprüche 1 bis 15 in einer oder für die Herstellung einer kosmetischen oder dermatologischen Zusammensetzung für den Schutz der Haut vor freien Radikalen.

**22.** Verfahren zur nicht-therapeutischen Behandlung der Haut, das für die Abschuppung der Haut vorgesehen ist, das darin besteht, auf die menschliche Haut eine Zusammensetzung aufzutragen, die eine wirksame Menge mindestens einer Verbindung nach einem der Ansprüche 1 bis 15 in einem kosmetisch und/oder dermatologisch akzeptablen Medium enthält.

**23.** Verfahren zur nicht-therapeutischen Behandlung der Alterung der menschlichen Haut, das darin besteht, auf die Haut eine Zusammensetzung aufzutragen, die eine wirksame Menge mindestens einer Verbindung nach einem der Ansprüche 1 bis 15 in einem kosmetisch und/oder dermatologisch akzeptablen Medium enthält.

**24.** Kosmetische und/oder dermatologische Zusammensetzung, dadurch gekennzeichnet, daß sie in einem kosmetisch oder dermatologisch akzeptablen Träger mindestens eine Verbindung nach einem der Ansprüche 1 bis 15 enthält.

**25.** Zusammensetzung nach Anspruch 24, dadurch gekennzeichnet, daß sie in Form einer Creme zum Schutz, zur Behandlung oder zur Pflege der Haut oder der Schleimhäute für das Gesicht, die Hände oder den Körper, einer Körpermilch zum Schutz oder zur Pflege, einer Lotion, eines Gels oder eines Schaums für die Pflege oder die Behandlung der Haut und der Schleimhäute oder für die Reinigung der Haut, einer Seife oder eines Reinigungsstücks vorliegt.

**26.** Zusammensetzung nach Anspruch 24 oder 25, dadurch gekennzeichnet, daß sie außerdem mindestens eine Verbindung enthält, die unter den Antagonisten der P-Substanz und/oder von CGRP (Calcitonin Gene Related Peptide oder Peptid, das mit dem Calcitonin-Gen verbunden ist), enthält.

**27.** Zusammensetzung nach einem der Ansprüche 24 bis 26, dadurch gekennzeichnet, daß sie 0,2 bis 20 Gew.-%, bezogen auf das Gesamtgewicht der Zusammensetzung, einer Verbindung nach einem der Ansprüche 1 bis 15 enthält.

## Claims

**1.** Product corresponding to one of formulae (1) to (3) below:

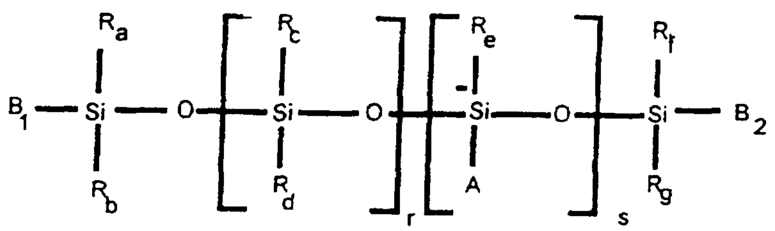

**(1)**

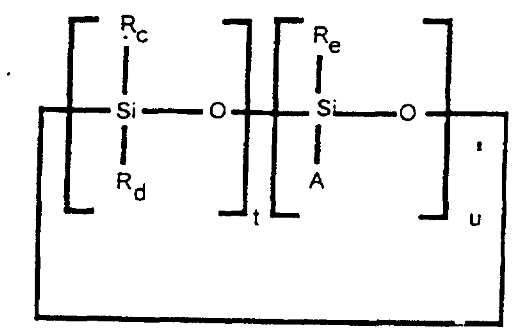

**(2)**

$$\mathbf{A\text{-}SiR'_1R'_2R'_3} \qquad (3)$$

in which:

- Ra to Rg, which are identical or different, are chosen from the group consisting of linear or branched, saturated or unsaturated $C_1$-$C_{10}$alkyl and $C_2$-$C_{10}$alkenyl radicals, the phenyl radical and the 3,3,3-trifluoropropyl radical, at least 80%, on a number basis, of the radicals Ra to Rg being the methyl radical.
- A denotes a monovalent radical attached directly to a silicon atom and corresponding to formula (4):

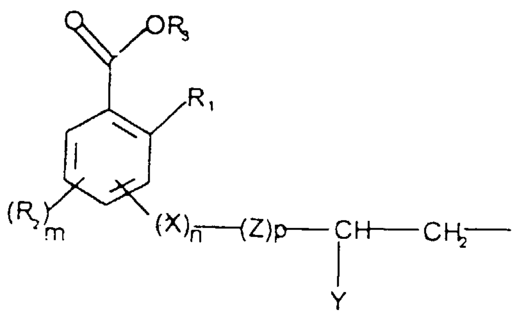

(4)

in which:

$R_1$ represents OH, a linear or branched, saturated or unsaturated $C_1$-$C_4$alkoxyl radical or an acyloxy function of formula $O(C{=}O)R_4$, in which $R_4$ represents a linear or branched $C_1$-$C_8$alkyl or $C_2$-$C_8$alkenyl radical,
$R_2$, which are identical or different, represent an OH radical, a linear or branched $C_1$-$C_8$alkyl or $C_2$-$C_8$alkenyl radical or a linear or branched $C_1$-$C_8$alkoxyl radical, it being possible for two adjacent $R_2$ radicals together to form an alkanedioxy group in which the alkane chain consists of 1 or 2 carbon atoms,
$R_3$ represents a radical chosen from: a hydrogen atom, a pharmaceutically acceptable cation, a linear or branched $C_1$-$C_6$alkyl or $C_2$-$C_6$alkenyl radical and a benzyl radical, optionally substituted with a group chosen from the following radicals: linear or branched $C_1$-$C_6$alkyl or $C_2$-$C_6$alkenyl, hydroxyl, amino, linear or branched $C_1$-$C_6$alkoxy or $C_2$-$C_6$alkenyloxy, halocarboxylic acid or linear or branched $C_1$-$C_6$alkylcarboxylate or $C_2$-$C_6$alkenyl carboxylate,
when $R_1$ is other than OH, $R_3$ is H or a pharmaceutically acceptable cation,
m is chosen from the integers 0, 1 and 2,
p is chosen from the integers 0 and 1,
X represents O, NH, C=O, NH(C=O)NH, NH(C=O) or (C=O)NH,
n is chosen from the integers 0 and 1,
Z is a linear or branched, saturated or unsaturated $C_1$-$C_6$alkanediyl radical, optionally substituted with a hydroxyl radical or a linear or branched, saturated or unsaturated $C_2$-$C_8$alkoxyl radical,
Y represents a hydrogen atom, a hydroxyl radical or a linear or branched, saturated or unsaturated $C_2$-$C_8$alkoxyl radical,

- $B_1$ and $B_2$, which are identical or different, are chosen from the radicals Ra to Rg and A defined above,
- $R'_1$, $R'_2$ and $R'_3$, which are identical or different, are chosen from linear or branched, saturated or unsaturated $C_1$-$C_8$alkyl and $C_2$-$C_8$alkenyl radicals, the phenyl radical and linear or branched, saturated or unsaturated $C_1$-$C_4$alkoxyl radicals,
- r is an integer ranging from 0 to 50,
- s is an integer ranging from 0 to 20, it being understood that if s is zero, then at least one of the radicals $B_1$ and $B_2$ denotes the radical A,
- u is an integer ranging from 1 to 6 and t is an integer ranging from 0 to 10, it being understood that t+u is greater than or equal to 3.

**2.** Product according to Claim 1, characterized in that, in formula (4), the chain unit $-(X)_n-Z-$ is attached to the aromatic ring in position 3, 4 or 5.

**3.** Product according to either of the preceding claims, characterized in that the radicals Ra to Rg are chosen from methyl, ethyl, propyl, n-butyl, n-octyl and 2-ethylhexyl radicals.

**4.** Product according to the preceding claim, characterized in that the radicals Ra to Rg represent methyl.

**5.** Product according to any one of the preceding claims, characterized in that the radicals $R'_1$, $R'_2$ and $R'_3$ are chosen from methyl, ethyl, propyl, n-butyl, n-octyl and 2-ethylhexyl radicals.

**6.** Product according to the preceding claim, characterized in that the radicals $R'_1$, $R'_2$ and $R'_3$ represent methyl.

**7.** Product according to any one of the preceding claims, characterized in that the alkyl radicals $B_1$ and $B_2$ are chosen from methyl, ethyl, propyl, n-butyl, n-octyl and 2-ethylhexyl radicals.

**8.** Product according to the preceding claim, characterized in that the radicals $B_1$ and $B_2$ both represent methyl.

**9.** Product according to any one of the preceding claims, characterized in that r is an integer ranging from 0 to 3.

**10.** Product according to any one of the preceding claims, characterized in that s is an integer ranging from 0 to 3.

**11.** Product according to any one of the preceding claims, characterized in that $R_1$ is OH.

**12.** Product according to any one of the preceding claims, characterized in that $R_3$ is a hydrogen atom.

**13.** Product according to any one of the preceding claims, characterized in that m = 0.

**14.** Product according to any one of the preceding claims, characterized in that n = 0 or n = 1 and X represents O.

**15.** Product according to any one of the preceding claims, characterized in that it is chosen from:

- methyl 2-hydroxy-4-(3-trimethylsilanylpropyloxy)benzoate,
- 2-hydroxy-4-(3-trimethylsilanylpropyloxy)benzoic acid,
- methyl 2-hydroxy-5-(3-trimethylsilanylpropyloxy)benzoate,
- 2-hydroxy-5-(3-trimethylsilanylpropyloxy)benzoic acid,
- methyl 2-hydroxy-4-[2-methyl-3-[1,3,3,3-tetramethyl-1-[(trimethylsilyl)oxy]disiloxanyl]propyloxy]benzoate,
- methyl 2-hydroxy-3-[2-methyl-3-[1,3,3,3-tetramethyl-1-[(trimethylsilyl)oxy]disiloxanyl]propyl]benzoate,
- 2-hydroxy-4-[2-methyl-3-[1,3,3,3-tetramethyl-1-[(trimethylsilyl)oxy]disiloxanyl]propyl]benzoic acid,
- 2-hydroxy-3-[2-methyl-3-[1,3,3,3-tetramethyl-1-[(trimethylsilyl)oxy]disiloxanyl]propyl]benzoic acid,
- 2-hydroxy-5-[3-[1,3,3,3-tetramethyl-1-[(trimethylsilyl)oxy]disiloxanyl]propylamido]benzoic acid.

**16.** Use of a product according to any one of the preceding claims in, or for the manufacture of, a cosmetic or dermatological composition to promote desquamation of human skin and/or to stimulate epidermal renewal.

**17.** Use of a product according to any one of Claims 1 to 15 to treat intrinsic and extrinsic ageing of human skin.

**18.** Use of a product according to any one of Claims 1 to 15, in, or for the manufacture of, a cosmetic or dermatological composition as an anti-ageing agent, to combat wrinkles and/or fine lines and/or actinic blemishes and/or skin dyschromias and/or dermatitis and/or scars on human skin.

**19.** Use of a product according to any one of Claims 1 to 15 in, or for the manufacture of, an antibacterial cosmetic or dermatological composition.

**20.** Use of a product according to any one of Claims 1 to 15 in, or for the manufacture of, an antisun cosmetic or dermatological composition.

**21.** . Use of a product according to any one of Claims 1 to 15 in, or for the manufacture of, a cosmetic or dermatological composition for protecting human skin against free radicals.

**22.** Non-therapeutic treatment process for the skin intended to desquamate the skin, which consists in applying to human skin a composition containing an effective amount of at least one product according to any one of Claims 1 to 15 in a cosmetically and/or dermatologically acceptable medium.

**23.** Process for the non-therapeutic treatment of the ageing of human skin, which consists in applying to this skin a composition containing an effective amount of at least one product according to any one of Claims 1 to 15 in a cosmetically and/or dermatologically acceptable medium.

**24.** Cosmetic and/or dermatological composition, characterized in that it comprises, in a cosmetically or dermatologically acceptable support, at least one product according to any one of Claims 1 to 15.

**25.** Composition according to Claim 24, characterized in that it is in the form of a protective, treatment or care cream for the skin or the mucous membranes, for the face, for the hands or for the body, a protective or care body milk, a care or treatment lotion, gel or foam for the skin and the mucous membranes or for cleansing the skin, a soap or a cleansing bar.

**26.** Composition according to Claim 24 or 25, characterized in that it also comprises at least one product chosen from antagonists of substance P and/or of CGRP (Calcitonin Gene Related Peptide).

**27.** Composition according to any one of Claims 24 to 26, characterized in that it comprises 0.2 to 20% by weight, relative to the total weight of the composition, of a product according to one of Claims 1 to 15.